# EUROPEAN PATENT APPLICATION

(11) **EP 4 420 669 A1**
(43) Date of publication of application: **28.08.2024**
(21) Application number: 22883407.3
(22) Date of filing: 11.10.2022
(51) Int. Cl.: A61K 36/07, A01N 65/00, A01P 1/00, A01P 3/00, A61P 31/10

(54) **PRODUCTION METHOD FOR WATER-SOLUBLE EXTRACT OF SPENT MUSHROOM BED USED FOR MUSHROOM CULTIVATION , BIOFILM INHIBITOR, PRODUCTION METHOD FOR BIOFILM INHIBITOR, AND BIOFILM INHIBITION METHOD**

(30) Priority: 18.10.2021 JP 2021170089
(71) Applicant: Environment Energy Co., Ltd., Fukuyama-shi, Hiroshima 721-0952 (JP)
(72) Inventor: KASHIMURA, Koji, Fukuyama-shi, Hiroshima 721-0952 (JP); NODA; Shuji, Fukuyama-shi, Hiroshima 721-0952 (JP)
(74) Representative: Dehns
(86) International application number: PCT/JP2022/037824
(87) International publication number: WO 2023/068103

(57) **Abstract**

The present invention relates to a method for producing a water-soluble extract of a spent mushroom bed after mushroom bed cultivation, the method including extracting a water-soluble extract by bringing a spent mushroom bed after mushroom bed cultivation into contact with pressurized water at a temperature of 32 to 64°C under a pressure equal to or higher than the saturated vapor pressure, wherein the water-soluble extract is capable of inhibiting a biofilm formed by Candida albicans. This provides: a method for producing a water-soluble extract of a spent bed after mushroom cultivation that can produce a water-soluble extract capable of effectively inhibiting biofilms formed by Candida albicans; a biofilm inhibitor; a method for producing a biofilm inhibitor; and a biofilm inhibition method.

## Description

### Technical Field

The present invention relates to a method for producing a water-soluble extract of a spent mushroom bed after mushroom cultivation that can produce a water-soluble extract capable of inhibiting biofilms formed by Candida albicans, a biofilm inhibitor, a method for producing a biofilm inhibitor, and a biofilm inhibition method.

### Background Art

In recent years, spent mushroom beds after mushroom cultivation of shiitake mushrooms and other mushrooms have been reused. For example, Patent Document 1 describes that mushroom components such as glucan and chitin are extracted by bringing pressurized hot water at a temperature of 110 to 250°C into contact with a spent bed after mushroom cultivation. Patent Document 2 proposes a composition for caries prevention containing as an active ingredient a water-soluble extracted component derived from mushrooms contained in waste liquid of shiitake mushroom cultivation and the like.

### Prior Art Document

### Patent Document

Patent Document 1: JP-A-2006-176765
Patent Document 2: JP-A-2010-77028

### Summary of Invention

### Problem to be Solved by the Invention

Patent Document 1, however, does not consider the use of extracted components from spent mushroom beds after mushroom cultivation to inhibit biofilms formed by oral microorganisms.

Although Patent Document 2 examines the inhibition of biofilms formed by cariogenic bacteria such as Streptococcus mutans and Streptococcus sobrinus, it does not examine the inhibition of biofilms formed by Candida albicans, which is a pathogenic fungus.

The present invention provides a method for producing a water-soluble extract of a spent mushroom bed after mushroom cultivation that can produce a water-soluble extract capable of effectively inhibiting biofilms formed by Candida albicans, a biofilm inhibitor, a method for producing a biofilm inhibitor, and a biofilm inhibition method.

### Means to Solve the Problem

The present invention relates to a method for producing a water-soluble extract of a spent mushroom bed after mushroom bed cultivation, the method including extracting a water-soluble extract by bringing a spent mushroom bed after mushroom bed cultivation into contact with pressurized water at a temperature of 32 to 64°C under a pressure equal to or higher than the saturated vapor pressure, wherein the water-soluble extract is capable of inhibiting a biofilm formed by Candida albicans.

The present invention relates to a biofilm inhibitor that inhibits a biofilm formed by Candida albicans, wherein the biofilm inhibitor contains a water-soluble extract of a spent mushroom bed after mushroom bed cultivation, wherein the water-soluble extract is extracted by bringing a spent mushroom bed after mushroom bed cultivation into contact with pressurized water at a temperature of 32 to 64°C under a pressure equal to or higher than the saturated vapor pressure.

The present invention relates to a biofilm inhibition method, the method including extracting a water-soluble extract by bringing a spent mushroom bed after mushroom bed cultivation into contact with pressurized water at a temperature of 32 to 64°C under a pressure equal to or higher than the saturated vapor pressure, and treating a biofilm of Candida albicans using the obtained water-soluble extract.

The present invention relates to method for producing a biofilm inhibitor, wherein the biofilm inhibitor contains a water-soluble extract of a spent mushroom bed after mushroom bed cultivation, the method including extracting a water-soluble extract by bringing a spent mushroom bed after mushroom bed cultivation into contact with pressurized water at a temperature of 32 to 64°C under a pressure equal to or higher than the saturated vapor pressure.

### Effect of the Invention

With the method for producing a water-soluble extract of a spent bed after mushroom cultivation according to the present invention, a water-soluble extract that can effectively inhibit a biofilm formed by Candida albicans can be obtained.

With the present invention, a biofilm inhibitor that can effectively inhibit a biofilm formed by Candida albicans can be provided.

With the biofilm inhibition method according to the present invention, a biofilm formed by Candida albicans can be effectively inhibited.

### Brief Description of Drawings

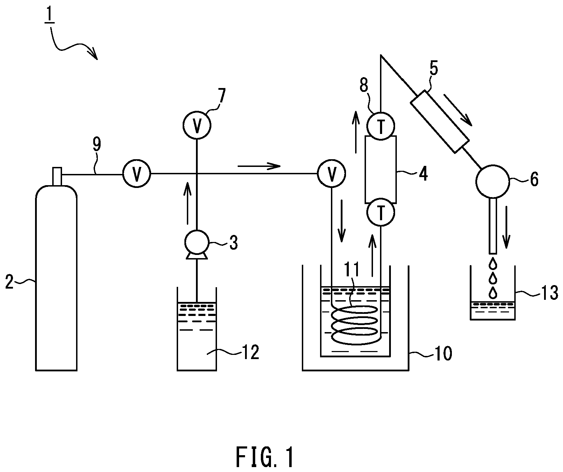

[Fig. 1] Fig. 1 is a schematic cross-sectional view of an example of a pressurized hot water treatment device.

### Description of Embodiments

The inventors of the present invention have repeatedly investigated the inhibitory effect of components derived from spent mushroom beds after mushroom cultivation on biofilms formed by Candida albicans, using spent mushroom beds after mushroom cultivation as a material. As a result, a water-soluble extract extracted under conditions different from those of conventional pressurized hot water treatment of a spent mushroom bed after mushroom cultivation, specifically, extracted by bringing a spent mushroom bed after mushroom bed cultivation into contact with pressurized water at a temperature of 32 to 64°C under a pressure equal to or higher than the saturated vapor pressure has a high inhibitory effect on biofilms formed by Candida albicans, specifically an effect of reducing the biofilm survival rate. In the following, unless otherwise specified, a "biofilm" means a biofilm formed by Candida albicans.

The spent bed after mushroom bed cultivation is not particularly limited, and any mushroom bed after harvesting mushrooms grown in the mushroom bed may be used. Mushrooms grown in a mushroom bed are not particularly limited, and may be white-rot fungi or brown-rot fungi. However, from the viewpoint of further increasing the biofilm inhibition effect of the water-soluble extract from a spent mushroom bed after mushroom cultivation, the spent mushroom bed is preferably a spent mushroom bed after cultivation of white-rot fungi. Examples of white-rot fungi include, but are not limited to, shiitake mushrooms, nameko mushrooms, maitake mushrooms, eryngii mushrooms, oyster mushrooms, and bunashimeji mushrooms. The spent mushroom bed is preferably a spent mushroom bed after cultivation of shiitake mushrooms, from the viewpoint of further increasing the biofilm inhibition effect of the water-soluble extract.

The mushroom bed may be a spent mushroom bed after all surface cultivation, or a spent mushroom bed after top surface cultivation. The number of times of cultivation is not particularly limited, and may be, for example, 1 to 8 times. The medium substrate material for the mushroom bed is not particularly limited, and any of oak, beech, Japanese cedar, cedar, etc. may be used as the raw material for wood chips, and any of bran, starch, rice bran, cornstarch, calcium carbonate, fossil seashells, shell powder, etc. may be used as the nutrient materials.

Before the spent mushroom bed is brought into contact with pressurized water, the spent mushroom bed may be cut or crushed into a predetermined size, for example, from the viewpoint of increasing the extraction effect of water-soluble extracts. The size of the spent mushroom bed after cutting or crushing is not particularly limited, but it is preferable to use one that consumes as little energy as possible. For example, the size may be several centimeters or less, and the spent mushroom bed is desirably in powder of a particle size of about 100 µm or less. Specifically, powder thereof screened to about 50 to 100 mesh may be used. Incidentally, the spent mushroom bed may be dried before being cut or crushed.

The treatment with pressurized water is carried out at a temperature of 32 to 64°C under a pressure equal to or higher than the saturated vapor pressure. It is assumed that by treating the spent mushroom bed with pressurized water under such conditions, a water-soluble extract containing many components that are highly effective in inhibiting a biofilm of Candida albicans can be obtained, and the effect of inhibiting a biofilm of Candida albicans can be increased.

From the viewpoint of further enhancing the biofilm inhibition effect of the water-soluble extract, the temperature of the pressurized water is preferably 60°C or lower, more preferably 57°C or lower, and even more preferably 52°C or lower.

The pressure of the pressurized water is not particularly limited as long as it is equal to or higher than the saturated vapor pressure, and may be, for example, 0.1 to 3.0 MPa or 0.1 to 2.5 MPa.

A water-soluble extract can be obtained by bringing a spent mushroom bed into contact with water in a sealed pressure-resistant container at the above-mentioned temperature and pressure. Extraction of the water-soluble extract can be carried out, for example, by bringing the spent mushroom bed into contact with water having a weight 10 to 100 times the weight of the spent mushroom bed.

The water-soluble extract is not particularly limited, and can be produced using, for example, a circulating pressurized water device. As the circulating pressurized water device, a known pressurized hot water treatment device can be used, such as the pressurized hot water treatment device shown in Fig. 1. The pressurized hot water treatment device 1 includes a nitrogen gas container 2, a high-pressure pump 3, a reactor 4, a cooler 5, a pressure holding valve 6, a valve 7, a thermocouple 8 and piping 9 connecting these, an oil bath 10, an external heat-insulating device (not shown), meters (not shown), and a treated liquid receiver 13. "11" indicates a heating coil, and "12" indicates water. The upper and lower ends of the reactor can be closed, for example, with porous filters. The porous filter is not particularly limited, but may have penetrating pores in a range of 2 to 100 µm, for example. The material of the porous filter may be metal such as stainless steel, or ceramic. With the size of the spent mushroom bed after cutting or crushing being taken into consideration, porous filters with the most suitable pores may be selected. For example, when the spent mushroom bed after crushing and drying is screened to about 50 to 100 mesh, gasket filters with an average pore size of about 5 µm may be used.

After the reactor is filled with a predetermined amount of a spent mushroom bed in a dry state, the reactor is connected to the piping, and then an external heat-insulating device is connected to the reactor and surrounding piping. Then, the system is filled with nitrogen gas, and the system internal pressure is adjusted to a predetermined pressure (for example, 0.1 to 3.0 MPa, or 0.1 to 2.5 MPa) using the pressure holding valve. Next, water is passed through the system using a high-pressure pump until it reaches to a point immersed in the oil bath, and the water flow is temporarily stopped. Then, the temperature of the inside of the system is controlled by the oil bath. Tap water may be used as the water, but water sufficiently purified, such as ion-exchange water, distilled water, or ultrafiltered water is desirably used. After the treated liquid having passed through the reactor is cooled to room temperature, the treated liquid that had been at a temperature range of 32 to 64°Cis collected at fixed time intervals (for example, every 5 minutes), whereby a water-soluble extract is obtained.

From the viewpoint of productivity, the water-soluble extract is desirably produced using a continuous-type apparatus. From the viewpoint of cost and ease of handling, the water-soluble extract can also be produced using a batch-type apparatus in which the temperature and pressure of water are adjusted within predetermined ranges.

The obtained water-soluble extract in an aqueous solution form may be dehydrated and dried by a known method such as freeze drying so as to be changed into a powder state. By making it into a powder state, storage stability is improved and transportation costs can be reduced.

The effect of inhibiting a biofilm of the water-soluble extract can be confirmed by doing the following: forming a biofilm of Candida albicans, and washing the film with a sterile buffer such as PBS to remove the floating Candida albicans; adding a water-soluble extract at a predetermined concentration to the biofilm, and subjecting the same to 24-hour stationary culture at 37°C; and thereafter, performing n XTT-reduction assay. The water-soluble extract can be adjusted using a medium (for example, RPMI1640 medium) so as to form a solution at a predetermined concentration. The solution may contain about 100 ppm to 3% ethanol, etc., if necessary. By using the RPMI1640 medium and a plate made of a predetermined material (for example, polystyrene, etc.), germination of mycelium can be promoted, whereby a biofilm can be formed. The term "RPMI1640 medium" is an abbreviation, and specifically, it is "RPMI-1640 without sodium bicarbonate supplemented with L-glutamine and buffered with 165 mM morpholinepropanesulfonic acid to pH 7." The XTT-reduction assay is a measurement method for quantifying mitochondrial respiratory capacity of Candida albicans (Candida albicans metabolic activity). In the XTT-reduction assay, when the relative value to a control that does not contain a water-soluble extract (relative value of biofilm survival rate) is 50% or less, it can be determined that biofilm formation of Candida albicans can be inhibited. In the XTT-reduction assay,the relative value to a control that does not contain a water-soluble extract (relative value of biofilm survival rate) is preferably 46% or less, more preferably 40% or less, and even more preferably 36% or less.

The water-soluble extract of the spent mushroom bed after mushroom bed cultivation obtained as described above can be used as a biofilm inhibitor for inhibiting a biofilm formed by Candida albicans. The water-soluble extract is a mixture containing many components, such as components derived from decomposed mushroom bed components and water-soluble components derived from shiitake mycelium, and it is not practical to identify the components of a water-soluble extract, even using various analytical methods such as elemental analysis, Fourier transform infrared spectroscopy (FT-IR), ¹³C CP/PASS NMR, HP-SEC (molecular size distribution), and THM-GC/MS.

The biofilm inhibitor can be suitably used in oral care for the purpose of plaque control. For example, biofilm inhibitors can be incorporated into toothpastes, mouthwashes, denture cleaners, and the like. Specifically, biofilm inhibitors may be used in combination with stabilizers, bactericidal ingredients, anti-inflammatory ingredients, sodium fluoride, etc. to form dentifrices, mouth rinses, denture cleaners, and the like.

The water-soluble extract of the spent mushroom bed after mushroom bed cultivation obtained as above can be used to inhibit the biofilm formed by Candida albicans. Biofilms of Candida albicans can be inhibited by treating them with the water-soluble extracts. The water-soluble extract can be used by adjusting the solution to a predetermined concentration, for example, 0.625 to 5 mg/L, using a solvent such as water, an organic solvent, or a medium (for example, RPMI1640 medium). The solution may contain an organic solvent such as about 100 ppm to 3% ethanol, if necessary.

### Example

The present invention is described below further in detail with reference to examples. The present invention, however, is not limited to the examples described below.

### (Experiment Example 1)

### <Spent mushroom bed>

The method used a mushroom bed used for shiitake mushroom (Letina edodes) bed cultivation (all surface cultivation, Ueno Village Mushroom Center, Gunma Prefecture, shiitake fungus strain H607, the number of cultivation times: 1 time, medium substrate material composition for mushroom bed: oak chips as wood, as well as Special Bran, Hominy feed, and fossil seashells as nutrients), discarded after shiitake mushrooms were cultivated and fruiting bodies thereof were harvested. The spent mushroom bed was crushed and dried with an air flow dryer, and thereafter, was screened to about 50 to 100 mesh.

### <Pressurized water device>

A pressurized water treatment device illustrated in Fig. 1 was used as the pressurized water treatment. The pressurized water treatment device 1 includes a nitrogen gas container 2, a high-pressure pump 3, a reactor 4, a cooler 5, a pressure holding valve 6, a valve 7, a thermocouple 8 and piping 9 connecting these, an oil bath 10, a treated liquid receiver 13, an external heat-insulating device (not shown), and meters (not shown). "11" indicates a heating coil, and "12" indicates water. The reactor was a flow-through percolator type reactor (SUS316, 23.5 mm i.d. × 65 mm length, 28 mL) capped at both ends with gasket filters (average pore size: 5 µm).

### <Pressurized water treatment of spent mushroom bed>

Nine g of the spent mushroom bed in a dried state (dried spent mushroom bed) described above was accurately weighed and was filled into a 28 mL stainless steel reactor, which then was connected to piping. Next, an external heat-insulating device was connected to the reactor and surrounding piping, the system was filled with nitrogen gas, and the system internal pressure was adjusted to 2.5 MPa using a pressure holding valve. Furthermore, the temperature in the system was controlled using an oil bath, and water treatment was performed by passing distilled water through the reactor at a flow rate of 10 mL/min. The temperature was set at 30 to 170°C, and the temperature was raised continuously over 70 minutes. The treated liquid after passing through the reactor was cooled, and then, collected every 5 minutes, to obtain water-soluble extracts with sample numbers A1 to A5. Table 1 below shows the temperatures of pressurized water corresponding to the water-soluble extracts of sample numbers A1 to A5. Sample numbers A1 to A3 correspond to Examples, and sample numbers A4 to A5 correspond to Comparative Examples.

### (Experiment Example 2)

Water-soluble extracts of sample numbers B 1 to B5 were obtained in the same manner as that of Example 1 except that mushroom beds for shiitake mushroom bed cultivation (top surface cultivation method; obtained from Agriculture and Forestry Corporation Shinshiro Tsukude, shiitake fungus strain H607, number of cultivations: 8 times, medium substrate material composition for mushroom bed: oak chips as wood, as well as bran, starch, rice bran, corn starch, and calcium carbonate as nutrients) that were discarded after shiitake mushroom cultivation and harvest of fruiting bodies were used. Table 2 below shows the temperatures of pressurized water corresponding to the water-soluble extracts of sample numbers B1 to B5. Sample numbers B1 to B3 correspond to Examples, and sample numbers B4 to B5 correspond to Comparative Examples.

### (Reference Example 1)

At Agriculture and Forestry Corporation Shinshiro Tsukude , mushroom bed exudates (water-soluble components) were collected when spent mushroom beds of shiitake mushrooms (shiitake fungus strain H607) grown using the top surface cultivation method were discarded, and suspended substances were removed by centrifugation (6000 g, 15 minutes). Thereafter, the extracts were filtered through glass fiber filter paper (GB 140, Advantec) with a pore size of 0.4 µm, and membrane filters (mixed cellulose ester, Advantec) with pore sizes of 0.45 µm and 0.2 µm, whereby samples containing only water-soluble components were prepared.

### (Reference Example 2)

At Ueno Village Mushroom Center in Gunma Prefecture, mushroom bed exudates were collected when spent mushroom beds of shiitake mushrooms (shiitake fungus strain H607) grown using the top surface cultivation method were discarded, and suspended substances were removed by centrifugation (6000 g, 15 minutes). Thereafter, the extracts were filtered through glass fiber filter paper (GB140, Advantec) with a pore size of 0.4 µm, and membrane filters (mixed cellulose ester, Advantec) with pore sizes of 0.45 µm and 0.2 µm, whereby samples containing only water-soluble components were prepared.

**[Table 1]**

| | Ex.1 | Ex. 2 | Ex. 3 | Comp. Ex. 1 | Comp. Ex. 2 |
|---|---|---|---|---|---|
| Sample No. | A1 | A2 | A3 | A4 | A5 |
| Temperature (°C) | Above 32, 38.5 or below | Above 38.5, 47.6 or below | Above 47.6, 57 or below | Above 57, 67.7 or below | Above 67.7,79.1 or below |

**[Table 2]**

| | Ex. 4 | Ex. 5 | Ex. 6 | Comp. Ex. 3 | Comp. Ex. 4 |
|---|---|---|---|---|---|
| Sample No. | B1 | B2 | B3 | B4 | B5 |
| Temperature (°C) | Above 33, 40 or below | Above 40, 52 or below | Above 52, 64 or below | Above 64, 76 or below | Above 76, 87 or below |

Regarding the water-soluble extracts of the spent mushroom beds of sample numbers A1 to A5 and B1 to B5 and the water-soluble components in the mushroom bed exudates of Reference Examples 1 to 2, inhibitory effects thereof on biofilm formation by Candida albicans were measured and evaluated as follows. The results are shown in Tables 3 and 4 below.

### (Inhibition on biofilms of Candida albicans)

### <Formation of biofilm>

Candida albicans SC5314 strain precultured on Sabouraud agar medium (37°C, overnight) was further cultured with shaking in YPD broth (30°C, 13 hours, 150 rpm) to obtain a test bacterial solution. The final inoculum concentration of this bacterial solution was adjusted to 1 × 10⁶ cells/mL, 100 µL of the same was dispensed into each well of a 96-well microtiter plate (flat bottom, made of polystyrene), and was subjected to stationary culture at 37°C for 24 hours, whereby a biofilm was formed. After that, the supernatant was removed, and each sample (containing 1% ethanol) was adjusted to 0.5 w/v% (approximately 200-fold dilution, concentration of water-soluble extract: 5 mg/mL) using RPMI1640 medium. Each sample, 100 µL, was inoculated into each well. In Examples and Comparative Examples, RPMI1640 medium containing 1% ethanol was used as a control. After a stationary culture at 37°C was further performed for 24 hours, the supernatant was removed, washed with PBS (pH 7.4, 10 mM), and subjected to XTT-reduction assay. In the case of Reference Examples 1 and 2, it was diluted 2 times using RPMI1640 medium. In Reference Examples, RPMI1640 medium was used as a control.

### <XTT-reduction assay>

XTT (2,3-bis[2-methoxy-4-nitro-5-sulfophenyl]-2H-tetrazolium-5-carboxyanilide, CAYMAN CHEMICAL COMPANY) was dissolved in sterile PBS (pH 7.4, 10mM), and 0.5mg/mL solution of the same was prepared. The solution was sterilized by filtration using a 0.22 µm filter. Menadione (adjusted to 10 mM with acetone) was added so that the solution had a final concentration of 1 µM. After the biofilm was washed, 100 µL of the XTT solution was dispensed into each well. After being left to stand at 37°C in the dark for 2 hours, 80 µL of the supernatant of the same was transferred to a new well, and an absorbance at 492 nm was measured using a microtiter plate reader.

The absorbance of the control was assumed to be 100%, and a relative value of the absorbance of each sample with respect to the absorbance of the control was determined. Average values and standard deviations (SD) of the relative absorbance values are shown regarding Examples in which n was 8, and Comparative Examples and Reference Examples in which n was 4.

**[Table 3]**

| Ex. | | 1 | 2 | 3 | 4 | 5 | 6 |
|---|---|---|---|---|---|---|---|
| Relative value of absorbance | Average value (%) | 30.7 | 35.4 | 33.9 | 31.1 | 38.8 | 45.6 |
| | SD (%) | 2.0 | 2.5 | 1.9 | 2.2 | 3.0 | 2.4 |

**[Table 4]**

| | | Comp. Ex. | | | | Ref. Ex. | |
|---|---|---|---|---|---|---|---|
| | | 1 | 2 | 3 | 4 | 1 | 2 |
| Relative value of absorbance | Average value (%) | 64.3 | 72.4 | 75.2 | 83.3 | 74.1 | 57.3 |
| | SD (%) | 2.1 | 5.3 | 3.9 | 2.0 | 3.1 | 3.7 |

As can be seen from the data in Table 3, in Examples 1 to 6 in which water-soluble extracts were extracted by bringing the spent mushroom bed after mushroom bed cultivation into contact with pressurized water at a temperature of 32 to 64°C under a pressure equal to or higher than the saturated vapor pressure, the obtained water-soluble extracts inhibited biofilm formation of Candida albicans to 50% or less (relative value of biofilm survival rate), which indicates that the water-soluble extracts were capable of effectively inhibiting the biofilm formed by Candida albicans.

On the other hand, the water-soluble extracts of Comparative Examples 1 to 4, which contained water-soluble extracts extracted by bringing the spent mushroom beds after mushroom bed cultivation into contact with pressurized water above 64°C, showed relative values of the biofilm survival rate of Candida albicans of more than 60%, which indicates that the extracts were unable to effectively inhibit the biofilm formed by Candida albicans.

Also, the water-soluble extracts of Reference Examples 1 to 2, obtained from mushroom bed exudates when spent mushroom beds used in the top surface cultivation method were discarded, had lower effects of inhibiting biofilms formed by Candida albicans than those of the water-soluble extracts obtained in Examples, even though the water-soluble extracts of Reference Examples 1 to 2 were used at high concentrations.

The present invention is not limited particularly, but encompasses the embodiments described below.
[1] A method for producing a water-soluble extract of a spent mushroom bed after mushroom bed cultivation, the method including:
   extracting a water-soluble extract by bringing a spent mushroom bed after mushroom bed cultivation into contact with pressurized water at a temperature of 32 to 64°C under a pressure equal to or higher than the saturated vapor pressure,
   wherein the water-soluble extract is capable of inhibiting the biofilm formed by Candida albicans.
[2] The method for producing a water-soluble extract according to [1], wherein the pressurized water has a pressure of 0.1 to 3.0 MPa and a temperature of 32 to 60°C.
[3] The method for producing a water-soluble extract according to [1] or [2], the method further including freeze-drying the water-soluble extract.
[4] The method for producing a water-soluble extract according to any one of [1] to [3], wherein the mushroom is a shiitake mushroom.
[5] A biofilm inhibitor for inhibiting biofilm formation by Candida albicans,
   the biofilm inhibitor containing a water-soluble extract of a spent mushroom bed after mushroom bed cultivation,
   wherein the water-soluble extract is extracted by bringing the spent mushroom bed after mushroom bed cultivation into contact with pressurized water at a temperature of 32 to 64°C under a pressure equal to or higher than the saturated vapor pressure.
[6] A biofilm inhibition method, the method including:
   extracting a water-soluble extract by bringing a spent mushroom bed after mushroom bed cultivation into contact with pressurized water at a temperature of 32 to 64°C under a pressure equal to or higher than the saturated vapor pressure; and
   treating a biofilm of Candida albicans with the obtained water-soluble extract.
[7] The biofilm inhibition method according to [6], wherein the pressurized water has a pressure of 0.1 to 3.0 MPa and a temperature of 35 to 60°C.
[8] The biofilm inhibition method according to [6] or [7], the method further including freeze-drying the water-soluble extract.
[9] The biofilm inhibition method according to any one of [6] to [8], wherein the mushroom is a shiitake mushroom.
[10] A method for producing a biofilm inhibitor,
   wherein the biofilm inhibitor contains a water-soluble extract of a spent mushroom bed after mushroom bed cultivation,
   the method including extracting the water-soluble extract by bringing the spent mushroom bed after mushroom bed cultivation into contact with pressurized water at a temperature of 32 to 64°C under a pressure equal to or higher than the saturated vapor pressure.
[11] The method for producing a biofilm inhibitor according to [10], wherein the pressurized water has a pressure of 0.1 to 3.0 MPa and a temperature of 32 to 60°C.
[12] The method for producing a biofilm inhibitor according to [10] or [11], the method further including freeze-drying the water-soluble extract.
[13] The method for producing a biofilm inhibitor according to any one of [10] or [12], wherein the mushroom is a shiitake mushroom.

### [Reference Signs List]

1: pressurized hot water treatment device 1
2: nitrogen gas container
3: high-pressure pump
4: reactor
5: cooler
6: pressure holding valve
7: valve
8: thermocouple
9: piping
10: oil bath
11: heating coil
12: water
13: treated liquid receiver

## Claims

1. A method for producing a water-soluble extract of a spent mushroom bed after mushroom bed cultivation, comprising:
extracting a water-soluble extract by bringing a spent mushroom bed after mushroom bed cultivation into contact with pressurized water at a temperature of 32 to 64°C under a pressure equal to or higher than saturated vapor pressure,
wherein the water-soluble extract is capable of inhibiting a biofilm formed by Candida albicans.

2. The method for producing a water-soluble extract according to claim 1,
wherein the pressurized water has a pressure of 0.1 to 3.0 MPa and a temperature of 32 to 60°C.

3. The method for producing a water-soluble extract according to claim 1 or 2, further comprising freeze-drying the water-soluble extract.

4. The method for producing a water-soluble extract according to any one of claims 1 to 3,
wherein the mushroom is a shiitake mushroom.

5. A biofilm inhibitor for inhibiting biofilm formation by Candida albicans, the biofilm inhibitor comprising a water-soluble extract of a spent mushroom bed after mushroom bed cultivation,
wherein the water-soluble extract is extracted by bringing the spent mushroom bed after mushroom bed cultivation into contact with pressurized water at a temperature of 32 to 64°C under a pressure equal to or higher than saturated vapor pressure.

6. A biofilm inhibition method, comprising:
extracting a water-soluble extract by bringing a spent mushroom bed after mushroom bed cultivation into contact with pressurized water at a temperature of 32 to 64°C under a pressure equal to or higher than saturated vapor pressure; and
treating a biofilm of Candida albicans with the water-soluble extract.

7. The biofilm inhibition method according to claim 6,
wherein the pressurized water has a pressure of 0.1 to 3.0 MPa and a temperature of 35 to 60°C.

8. The biofilm inhibition method according to claim 6 or 7, further comprising freeze-drying the water-soluble extract.

9. The biofilm inhibition method according to any one of claims 6 to 8,
wherein the mushroom is a shiitake mushroom.

10. A method for producing a biofilm inhibitor,
wherein the biofilm inhibitor contains a water-soluble extract of a spent mushroom bed after mushroom bed cultivation,
the method comprising extracting the water-soluble extract by bringing the spent mushroom bed after mushroom bed cultivation into contact with pressurized water at a temperature of 32 to 64°C under a pressure equal to or higher than the saturated vapor pressure.

11. The method for producing a biofilm inhibitor according to claim 10,
wherein the pressurized water has a pressure of 0.1 to 3.0 MPa and a temperature of 32 to 60°C.

12. The method for producing a biofilm inhibitor according to claim 10 or 11, further comprising freeze-drying the water-soluble extract.

13. The method for producing a biofilm inhibitor according to any one of claims 10 to 12,
wherein the mushroom is a shiitake mushroom.
